(19) Europäisches Patentamt European Patent Office Office européen des brevets

(11) **EP 3 780 974 B1**

(12) **EUROPÄISCHE PATENTSCHRIFT**

(45) Veröffentlichungstag und Bekanntmachung des Hinweises auf die Patenterteilung:
**12.11.2025 Patentblatt 2025/46**

(21) Anmeldenummer: **18720169.4**

(22) Anmeldetag: **17.04.2018**

(51) Internationale Patentklassifikation (IPC):
**A23L 33/105** (2016.01)   **A61K 36/61** (2006.01)

(52) Gemeinsame Patentklassifikation (CPC):
(C-Sets verfügbar)
**A23L 33/105; A61K 36/61;** A23V 2002/00   (Forts.)

(86) Internationale Anmeldenummer:
**PCT/EP2018/059743**

(87) Internationale Veröffentlichungsnummer:
**WO 2019/201425 (24.10.2019 Gazette 2019/43)**

(54) **GUAVEEXTRAKT**

GUAVA EXTRACT

EXTRAIT DE GOYAVE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**24.02.2021 Patentblatt 2021/08**

(73) Patentinhaber: **PM-International AG 5445 Schengen (LU)**

(72) Erfinder:
• **WEGHUBER, Julian 4591 Molln (AT)**
• **SCHWARZINGER, Bettina 4600 Wels (AT)**

(74) Vertreter: **Meissner Bolte Partnerschaft mbB Patentanwälte Rechtsanwälte Postfach 86 06 24 81633 München (DE)**

(56) Entgegenhaltungen:
CN-A- 104 984 186    CN-A- 106 107 993
CN-A- 108 114 024    JP-A- 2009 013 146
JP-A- 2013 107 897    US-A1- 2010 249 248

• **KONG K W ET AL: "Lycopene-rich fractions derived from pink guava by-product and their potential activity towards hydrogen peroxide-induced cellular and DNA damage", FOOD CHEMISTRY, ELSEVIER LTD, NL, vol. 123, no. 4, 15 December 2010 (2010-12-15), pages 1142 - 1148, XP027155713, ISSN: 0308-8146, [retrieved on 20100520]**

(52) Gemeinsame Patentklassifikation (CPC): (Forts.)

C-Sets
A23V 2002/00, A23V 2200/328, A23V 2300/14,
A23V 2300/44, A23V 2300/50

**Beschreibung**

Die Erfindung betrifft ein Verfahren zur Herstellung eines Extraktes aus Früchten der Echten Guave (Psidium Guajava). Ferner bezieht sich die Erfindung auf einen Extrakt, erhältlich durch das erfindungsgemäße Verfahren, und eine Zusammensetzung, enthaltend den erfindungsgemäßen Extrakt. Außerdem betrifft die vorliegende Erfindung die Verwendung der erfindungsgemäßen Zusammensetzung als Nahrungsergänzungsmittel und/oder Arzneimittel. Die Erfindung betrifft auch die Herstellung einer erfindungsgemäßen Zusammensetzung, insbesondere eines Nahrungsergänzungsmittels.

Aus der WO 2017/137405 ist bekannt, dass Guaijaverin (Quercetin-3-O-alpha-I-arabinopyranosid) eine Reduzierung und/oder Hemmung der intestinalen Glucose-Resorption bewirken kann. Guaijaverin ist ein Naturstoff aus der Echten Guave. Guaijaverin ist ferner ein Polyphenol und wird den Naturstoffen der Gruppe der Flavonole zugeordnet, an den Grundkörper Quercetin ist glykosidisch der Zucker Arabinose gebunden.

Extrakte aus der Echten Guave können aus Blättern, Früchten oder Samen der Echten Guave beispielsweise unter Einsatz organischer Lösungsmittel wie Ethanol oder Ethylacetat und bei erhöhten Temperaturen erhalten werden. Solche Extrakte unterscheiden sich je nach dem extrahierten Material, also Blättern, Früchten oder Samen, und dem Extraktionsverfahren in ihrer Zusammensetzung, enthalten aber häufig verschiedene Polyphenole wie Guaijaverin. Herkömmliche Extraktionsverfahren sind beispielsweise die Soxhlet-Extraktion, die Ultraschallextraktion und die Mazeration. Die US 2010/249248 A1 beschreibt alkoholische Extrakte aus der Frucht der Guave, die den Kohlenhydratmetabolismus positiv beeinflussen sollen.

In den letzten Jahren hat sich die Destraktion, auch Hochdruckextraktion, HDE, Fluidextraktion oder supercritical fluid extraction genannt, als neues Verarbeitungs- und Extraktionsverfahren in der Lebensmittelchemie etabliert. Bei der Destraktion wird ein Gas durch einen hohen Druck und eventuell erhöhte Temperaturen in den überkritischen Zustand überführt. Bei bestimmten Gasen tritt im überkritischen Zustand ein starker Anstieg des Lösevermögens für bestimmte Stoffe auf. Als Destraktionsmittel geeignete Gase sind beispielsweise Ethen, Propan, Ammoniak, Kohlenstoffdioxid, Distickstoffdioxid und Chlortrifluormethan.

Die Destraktion ist beispielsweise zur Abtrennung bestimmter schwerflüchtiger und thermisch instabiler Naturstoffe geeignet. Aus der bereits erwähnte Tatsache, dass bei bestimmten Gasen im überkritischen Zustand ein starker Anstieg des Lösevermögens für bestimmte Stoffe auftritt, resultiert, dass die Destraktion heute zur der Entcoffeinierung von Kaffee, zur Hopfen- und Gewürzexktraktion, zur Abtrennung von freien Fettsäuren aus Fetten und Ölen, zum Entfernen kleiner, mineralischer Partikel aus hochviskosen Flüssigkeiten, zur Trennung von Mono- und Diacylglycerolen und bei vielen weiteren Stofftrennungen eingesetzt wird. Auf Grundlage des Vermögens der superkritischen Flüssigkeiten, bestimmte Stoffe gezielt zu trennen, wurde außerdem die überkritische Flüssigkeitschromatographie (SFC) entwickelt.

Castro-Vargas H. I. et al. (J. of Supercritical Fluids 51, 2010, 319-324) beschreiben die Destraktion einer phenolischen Fraktion aus Samen der Echten Guave. Da die Extraktion mit reinem Kohlenstoffdioxid im Vergleich zu einer herkömmlichen ethanolischen Soxhlet-Extraktion sehr geringe Ausbeuten liefert, wird Ethanol bzw. Ethylacetat als Cosolvens verwendet. Durch das Cosolvens übertrifft die Destraktion die ethanolische Extraktion an Gesamtausbeute, der Anteil der phenolischen Fraktion an dem gewonnenen Extrakt bleibt allerdings geringer als bei der herkömmlichen ethanolischen Extraktion. Die chemische Analyse deutet an, dass sich der durch ethanolische Extraktion und der durch Destraktion gewonnen Extrakt in ihrer Zusammensetzung unterscheiden, da je nach Testmethode der durch ethanolische Extraktion oder der durch Destraktion gewonnen Extrakt in seiner antioxidativen Wirkung überlegen ist.

Moura P. M. et al. (J. of Supercritical Fluids 62, 2012, 116-122) beschreiben die Extraktion von Blättern der Echten Guave mittels Destraktion mit Kohlenstoffdioxid und bevorzugt Ethanol als Cosolvens. Gegenüber herkömmlichen Extrakten weisen die Kohlenstoffdioxid-Extrakte laut Moura P. M. et al. einen höheren Gehalt an essentiellen Ölen, Flavonoiden, antioxidativen Verbindungen und beta-Caroten auf. Auch die CN 104 984 186 A und CN 106 107 993 A betreffen Destraktionsextrakte aus der Guave.

Der vorliegenden Erfindung liegt die Aufgabe zu Grunde, ein Verfahren unter Verwendung der Destraktion zur Herstellung eines Extraktes aus Früchten der Echten Guave (Psidium Guajava) anzugeben. Eine weitere Aufgabe der Erfindung ist es, den durch das Verfahren erhältlichen Extrakt und eine Zusammensetzung, umfassend den Extrakt, anzugeben. Ebenso ist es Aufgabe der Erfindung, die Verwendung der Zusammensetzung als Nahrungsergänzungsmittel und/oder Arzneimittel und ein Verfahren zur Herstellung der Zusammensetzung, insbesondere eines Nahrungsergänzungsmittels, anzugeben.

Erfindungsgemäß wird diese Aufgabe durch ein Verfahren nach Anspruch 1 gelöst. Mit Blick auf den Extrakt, erhältlich durch das erfindungsgemäße Verfahren, und die Zusammensetzung, umfassend den erfindungsgemäßen Extrakt, wird diese Aufgabe durch Anspruch 10 und 11 gelöst. Bezüglich der Verwendung der erfindungsgemäßen Zusammensetzung wird diese Aufgabe durch Anspruch 12 gelöst. Bezüglich des Verfahrens zur Herstellung der erfindungsgemäßen Zusammensetzung, insbesondere eines Nahrungsergänzungsmittels, wird diese Aufgabe durch Anspruch 15 gelöst.

Die Erfindung beruht auf dem Gedanken, ein Verfahren zur Herstellung eines Extraktes aus Früchten der Echten Guave (Psidium Guajava) anzugeben. Bevorzugt umfasst das Verfahren ein Bereitstellen eines zu extrahierenden Materiales, hergestellt aus Früchten der Echten Guave, und eine Destraktion des zu extrahierenden Materiales mit Kohlenstoffdioxid als Destraktionsmittel in einer Hochdruckextraktionsanlage, um einen Extrakt zu erhalten.

Überraschenderweise wird durch das erfindungsgemäße Verfahren ein Extrakt erhalten, in welchem keines jener Polyphenole oder Flavonoide nachgewiesen wird, welche in herkömmlichen Extrakten der Echten Guave, beispielsweise einem ethanolischen Extrakt aus Früchten der Echten Guave oder einem Extrakt aus Blättern der Echten Guave, vorkommen. Somit unterscheidet sich die Zusammensetzung des erhaltenen Extraktes, insbesondere in Bezug auf Polyphenole, stark von der Zusammensetzung von Extrakten der Echten Guave, erhältlich durch nicht erfindungsgemäße Verfahren. Ungeklärt ist, ob bei dem vorliegenden Verfahren während der Destraktion nur ganz bestimmte Verbindungen extrahiert werden, oder ob zusätzlich durch ein möglicherweise vorliegendes, saures Gemisch aus überkritischem Kohlenstoffdioxid und Wasser neue Produkte gebildet werden, wobei die Destraktion also kein reines Extraktionsverfahren, sondern überraschenderweise auch ein neues Herstellungsverfahren ist. Noch überraschender ist, dass der erfindungsgemäße Extrakt eine viel stärkere Hemmung des Glucosetransportes, eine ausgeprägtere Reduzierung von Blutzuckerspitzen und eine stärkere Hemmung intestinaler Glucose-Resorption aufweist, als herkömmliche Vergleichsextrakte.

Bevorzugte Ausführungsformen der Erfindung sind in den Unteransprüchen angegeben.

In bevorzugten Ausführungsformen bezeichnet der Begriff Frucht bzw. Früchte Fruchtfleisch, optional mit Haut oder Schale.

Vorzugsweise umfasst der Begriff Frucht bzw. Früchte nicht die Samen, Kerne und/oder Blätter oder Teile davon.

Als Destraktionsmittel für das erfindungsgemäße Verfahren wird bevorzugt Kohlenstoffdioxid verwendet. Anstelle von Kohlenstoffdioxid kann in einem erfindungsgemäßen Verfahren als Destraktionsmittel beispielsweise auch Ethen, Propan, Ammoniak, Distickstoffdioxid, Distickstoffmonoxid und/oder Chlortrifluormethan eingesetzt werden.

In einer bevorzugten Ausführungsform umfasst die Hochdruckextraktionsanlage mindestens einen Extraktor. In dem Extraktor liegt das Kohlenstoffdioxid in überkritischem Zustand vor und extrahiert das zu extrahierende Material.

In einer bevorzugten Ausführungsform umfasst die Hochdruckextraktionsanlage mindestens einen Abscheider. In dem Abscheider herrscht eine niedrigere Temperatur und ein geringerer Druck als in dem Extraktor, wodurch sich aus Kohlenstoffdioxid, welches aus dem Extraktor in den Abscheider überführt wird, ein Extrakt abscheidet.

Bevorzugt erfolgt die Destraktion, indem einem Extraktor Kohlenstoffdioxid zugeführt wird und mit Extrakt beladenes Kohlenstoffdioxid aus dem Extraktor in einen Abscheider geführt wird, in welchem sich Extrakt aus dem Kohlenstoffdioxid abscheidet.

Bevorzugt erfolgt die Destraktion in einem kontinuierlichen Modus, in welchem dem Extraktor kontinuierlich reines Kohlenstoffdioxid zugeführt wird und kontinuierlich mit Extrakt beladenes Kohlenstoffdioxid aus dem Extraktor in den Abscheider geführt wird, in welchem sich Extrakt aus dem Kohlenstoffdioxid abscheidet.

In einer bevorzugten Ausführungsform umfasst das zu extrahierende Material ein Mus aus Früchten der Echten Guave oder einen Feststoff, erhalten durch Trocknung von Mus aus Früchten der Echten Guave.

In einer bevorzugten Ausführungsform umfasst das zu extrahierende Material zu mindestens 60 Gew.-% ein Mus aus Früchten der Echten Guave oder einen Feststoff, erhalten durch Trocknung von Mus aus Früchten der Echten Guave.

In einer bevorzugten Ausführungsform ist das zu extrahierende Material ein Mus aus Früchten der Echten Guave oder ein Feststoff, erhalten durch Trocknung eines Muses aus Früchten der Echten Guave.

Vorzugsweise enthält das zu extrahierende Material keine Samen, Kerne und/oder Blätter der Echten Guave oder Teile davon.

Vorzugsweise enthält das Mus aus Früchten der Echten Guave oder der Feststoff, erhalten durch Trocknung eines Muses aus Früchten der Echten Guave, keine Samen, Kerne und/oder Blätter der Echten Guave oder Teile davon.

Vorzugsweise wird das zu extrahierende Material vor der Destraktion auf einen Wassergehalt von 3 Gew.-% bis 50 Gew.-%, bevorzugt von 5 Gew.-% bis 35 Gew.-%, besonders bevorzugt von 7 Gew.-% bis 30 Gew.-%, getrocknet.

In einer bevorzugten Ausführungsform wird das zu extrahierende Material vor der Destraktion zu einem Feststoff mit einem Wassergehalt von 7 Gew.-% bis 30 Gew.-% getrocknet.

In einer bevorzugten Ausführungsform wird das zu extrahierende Material vor der Destraktion mittels Trockenschrank oder Vakuumkammer getrocknet.

In einer möglichen Ausführungsform wird das zu extrahierende Material vor der Destraktion bei 30 °C bis 75 °C, bevorzugt bei 35 °C bis 65 °C, über eine Dauer von 20 h bis 120 h, bevorzugt von 40 h bis 80 h, getrocknet.

In einer bevorzugten Ausführungsform wird das zu extrahierende Material vor der Destraktion bei 30 °C bis 50 °C in einer Vakuumkammer getrocknet.

Falls das zu extrahierende Material ein Feststoff, erhalten durch Trocknung eines Muses aus Früchten der Echten Guave, ist oder diesen Feststoff umfasst, wird der Feststoff vor der Destraktion bevorzugt zerkleinert. Bevorzugt wird der Feststoff zu einem Pulver zerkleinert, wobei bevorzugt mindestens 80 Gew.-% der Pulverbestandteile einen Durchmesser zwischen 0,05 mm und 30 mm, bevorzugt zwischen 0,1 mm und 10 mm, aufweisen.

Vorzugsweise wird dem zu extrahierenden Material für die Destraktion Wasser als Cosolvens zugesetzt, bevorzugt 1 Gew.-% bis 30 Gew.-% Wasser, besonders bevorzugt 8 Gew.-% bis 20 Gew.-% Wasser, bezogen auf die Trockenmasse des zu extrahierenden Materiales. Durch Wasser als Cosolvens lässt sich die Ausbeute der Destraktion steigern.

Vorzugsweise beträgt der Druck in einem Extraktor der Hochdruckextraktionsanlage während der Destraktion 100 bar bis 500 bar, bevorzugt 200 bar bis 400 bar, ganz besonders bevorzugt 250 bar bis 350 bar.

Vorzugsweise beträgt die Temperatur in einem Extraktor der Hochdruckextraktionsanlage während der Destraktion 31 °C bis 80 °C, bevorzugt 31 °C bis 50 °C.

In einer möglichen Ausführungsform erfolgt die Destraktion über 1 h bis 8 h, bevorzugt 2 h bis 4 h.

In einer bevorzugten Ausführungsform umfasst die Hochdruckextraktionsanlage einen Extraktor und einen Abscheider und der Extraktor wird während der Destraktion durch einen kontinuierlichen Fluss an Kohlenstoffdioxid von 2 bis 20, bevorzugt von 5 bis 12, Kilogramm Kohlenstoffdioxid pro Stunde pro Kilogramm zu extrahierenden Materiales, durchströmt, wobei der kontinuierliche Fluss an Kohlenstoffdioxid dem Abscheider zugeführt wird. Die Menge an Kohlenstoffdioxid bezieht sich auf die Trockenmasse des zu extrahierenden Materiales.

Vorzugsweise liegt in einem Abscheider der Hochdruckextraktionsanlage während der Destraktion eine Temperatur von 10 °C bis 40 °C, bevorzugt von 15 °C bis 35 °C, vor.

Vorzugsweise liegt in einem Abscheider der Hochdruckextraktionsanlage während der Destraktion ein Druck von 15 bar bis 55 bar, bevorzugt von 30 bar bis 50 bar, vor.

In einer bevorzugten Ausführungsform ist der Wassergehalt des zu extrahierenden Materiales zu Beginn der Destraktion auf 3 Gew.-% bis 50 Gew.-%, bevorzugt auf 7 Gew.-% bis 30 Gew.-%, eingestellt.

In einer bevorzugten Ausführungsform wird ein erhaltener Extrakt nach der Destraktion getrocknet, wobei die Trocknung bevorzugt mittels Trockenschrank oder Vakuumkammer erfolgt und wobei der Wassergehalt nach der Trocknung bevorzugt < 10 Gew.-% beträgt.

In einer bevorzugten Ausführungsform unterscheidet sich die Zusammensetzung des erhaltenen Extraktes, insbesondere in Bezug auf Polyphenole, stark von der Zusammensetzung von Extrakten der Echten Guave, erhältlich durch nicht erfindungsgemäße Verfahren.

In einer bevorzugten Ausführungsform wird in dem erhaltenen Extrakt keines jener Polyphenole identifiziert, welche in Extrakten der Echten Guave, erhältlich durch nicht erfindungsgemäße Verfahren, identifiziert werden, wobei die Polyphenole bevorzugt mit einer in den Beispielen genannten HPLC-Methode identifiziert werden.

In einer bevorzugten Ausführungsform wird in dem erhaltenen Extrakt keines jener Polyphenole identifiziert, welche in Extrakten der Echten Guave, erhältlich durch nicht erfindungsgemäße Verfahren, identifiziert werden, wobei die Polyphenole bevorzugt mit einer in den Beispielen genannten HPLC-Methode identifiziert werden, und wobei die Polyphenole Phloridzin, Phloretin, Quercetin, Quercitrin, Isoquercitrin, Hyperosid, Avicularin, Guaijaverin, Procyanidin B1 und B2, (+)-Catechin, (-)-Catechin, (-)-Epicatechin, Gallocatechin, Epicatechingallat, Gallussäure und Ellagsäure umfassen oder sind.

In einer bevorzugten Ausführungsform enthält der erhaltene Extrakt von den Verbindungen Phloridzin, Phloretin, Quercetin, Quercitrin, Isoquercitrin, Hyperosid, Avicularin, Guaijaverin, Procyanidin B1 und B2, (+)-Catechin, (-)-Catechin, (-)-Epicatechin, Gallocatechin, Epicatechingallat, Gallussäure und Ellagsäure jeweils weniger als 0,8 Gew.-%, bevorzugt jeweils weniger als 0,3 Gew.-%, bezogen auf die Trockenmasse des Extraktes.

Die Erfindung beruht ferner auf dem Gedanken, einen Extrakt, erhältlich durch das erfindungsgemäße Verfahren, anzugeben.

In einer bevorzugten Ausführungsform unterscheidet sich die Zusammensetzung des erhaltenen Extraktes, insbesondere in Bezug auf Polyphenole, stark von der Zusammensetzung von Extrakten der Echten Guave, erhältlich durch nicht erfindungsgemäße Verfahren.

In einer bevorzugten Ausführungsform wird in dem erhaltenen Extrakt keines jener Polyphenole identifiziert, welche in Extrakten der Echten Guave, erhältlich durch nicht erfindungsgemäße Verfahren, identifiziert werden, wobei die Polyphenole bevorzugt mit einer in den Beispielen genannten HPLC-Methode identifiziert werden.

In einer bevorzugten Ausführungsform wird in dem erhaltenen Extrakt keines jener Polyphenole identifiziert, welche in Extrakten der Echten Guave, erhältlich durch nicht erfindungsgemäße Verfahren, identifiziert werden, wobei die Polyphenole bevorzugt mit einer in den Beispielen genannten HPLC-Methode identifiziert werden, und wobei die Polyphenole Phloridzin, Phloretin, Quercetin, Quercitrin, Isoquercitrin, Hyperosid, Avicularin, Guaijaverin, Procyanidin B1 und B2, (+)-Catechin, (-)-Catechin, (-)-Epicatechin, Gallocatechin, Epicatechingallat, Gallussäure und Ellagsäure umfassen oder sind.

In einer bevorzugten Ausführungsform enthält der erhaltene Extrakt von den Verbindungen Phloridzin, Phloretin, Quercetin, Quercitrin, Isoquercitrin, Hyperosid, Avicularin, Guaijaverin, Procyanidin B1 und B2, (+)-Catechin, (-)-Catechin, (-)-Epicatechin, Gallocatechin, Epicatechingallat, Gallussäure und Ellagsäure jeweils weniger als 0,8 Gew.-%, bevorzugt jeweils weniger als 0,3 Gew.-%, bezogen auf die Trockenmasse des Extraktes.

Die Erfindung beruht ferner auf dem Gedanken, eine Zusammensetzung, enthaltend den durch das erfindungsgemäße Verfahren erhältlichen Extrakt, anzugeben.

Überraschenderweise wurden in dem erfindungsgemäßen Extrakt bzw. der Zusammensetzung keines der Polyphenole oder Flavonoide nachgewiesen, die in herkömmlichen Extrakten der Echten Guave, beispielsweise einem ethanolischen Extrakt aus Früchten der Echten Guave oder einem Extrakt aus Blättern der Echten Guave, vorkommen. Dies ist insbesondere überraschend, da der erfindungsgemäße Extrakt eine viel stärkere Hemmung des Glucosetransportes, eine ausgeprägtere Reduzierung von Blutzuckerspitzen und eine stärkere Hemmung intestinaler Glucose-Resorption aufweist, als herkömmliche Vergleichsextrakte.

Daher beruht die Erfindung auch auf dem Gedanken, einen Extrakt, erhältlich durch das erfindungsgemäße Verfahren, oder eine Zusammensetzung, enthaltend den durch das erfindungsgemäße Verfahren erhältlichen Extrakt, zur Verwendung als Nahrungsergänzungsmittel und/oder Arzneimittel anzugeben.

Insbesondere soll eine Zusammensetzung zur Verwendung zur Reduzierung von Blutzuckerspitzen und/oder zur Hemmung intestinaler Glucose-Resorption und/oder zur Gewichtsreduktion angegeben werden.

In bevorzugten Ausführungsformen handelt es sich um eine Zusammensetzung zur Verwendung bei der Behandlung oder Prävention von Diabetes mellitus und/oder Hyperglykämie und/oder Adipositas.

Die Erfindung beruht auch auf dem Gedanken, ein Verfahren zur Herstellung einer erfindungsgemäßen Zusammensetzung, insbesondere eines Nahrungsergänzungsmittels, enthaltend den erfindungsgemäßen Extrakt, anzugeben, wobei der Extrakt durch das erfindungsgemäße Verfahren erhältlich ist. Das Verfahren zur Herstellung einer erfindungsgemäßen Zusammensetzung umfasst:

a) Bereitstellen eines Extraktes, erhältlich durch das Verfahren nach einem der Ansprüche 1 bis 10;
b) Auftragen des Extraktes auf eine Trägersubstanz, um eine extrakthaltige Trägersubstanz zu erhalten;
c) Mischen der extrakthaltigen Trägersubstanz mit weiteren Bestandteilen der Zusammensetzung, insbesondere des Nahrungsergänzungsmittels.

Bevorzugt erfolgt das Auftragen des Extraktes in Form einer Lösung des Extraktes in wässrigem Ethanol oder Propanol.

Bevorzugt erfolgt das Auftragen des Extraktes durch Aufsprühen einer Lösung des Extraktes auf eine Trägersubstanz.

Bevorzugt wird die extrakthaltige Trägersubstanz nach dem Auftragen des Extraktes getrocknet, um Wasser und Alkohol zu entfernen.

Bevorzugt wird die Trägersubstanz während des Auftragens durchmischt.

Das Verfahren gewährleistet eine besonders gleichmäßige Verteilung des Extraktes in der Zusammensetzung.

Die einzelnen Schritte bei der Durchführung der Destraktion und dem Bereitstellen eines zu extrahierenden Materiales, hergestellt aus Früchten der Echten Guave, sowie die geeignete Hochdruckextraktionsanlage sind dem Fachmann bekannt. Auch das Bereitstellen eines Muses aus Früchten der Echten Guave ist dem Fachmann bekannt.

Falls nichts anderes angegeben ist, wird der Wassergehalt in der Patentanmeldung jeweils nach folgender Formel berechnet:

$$\text{Wassergehalt (Gew.-\%)} = 100 \times \text{Masse (Wasser)} / \text{Masse (Wasser + Trockenmasse)}$$

Die Erfindung wird nachfolgend anhand eines Ausführungsbeispiels unter Bezug auf die beigefügten schematischen Zeichnungen näher erläutert.

In diesen zeigen:

Fig. 1        ein HPLC-Chromatogramm eines erfindungsgemäßen Extraktes;

Fig. 2        die Wirkung eines erfindungsgemäßen Extraktes und mehrerer Vergleichsextrakte auf den Glucosetransport von Caco-2 Zellen;

Fig. 3A-3C     die Wirkung eines erfindungsgemäßen Extraktes und eines Vergleichsextraktes während eines Glucosetoleranztestes bei Mäusen.

Erfindungsgemäßes Beispiel betreffend ein Verfahren zur Herstellung eines Extraktes aus Früchten der Echten Guave

Als zu extrahierendes Material, hergestellt aus Früchten der Echten Guave, wird "Single Strength White Guava Puree" bezogen. Das Mus weist einen Grad von mindestens 8,5 Brix auf, stammt aus Indien und wird, in einer Spezialfolie eingeschweißt, in einem gekühlten Aseptikfass geliefert. Das Mus weist keine Samen, Kerne oder Blätter der Echten Guave oder Teile davon auf. Das Mus wird auf Silikonmatten aufgebracht und im Trockenschrank bei 60 °C und unter regelmäßigem Wenden für 72 Stunden getrocknet. Durch Trocknung des Muses aus Früchten der Echten Guave wird ein Feststoff mit einem Wassergehalt von etwa 23 Gew.-% erhalten. Das zu extrahierende Material wird in einem Eisfach auf -20 °C gekühlt und dann mit einem JTC Blender zerkleinert, wobei die meisten Teilchen einen Durchmesser zwischen 0,1 mm und 10 mm aufweisen. Das zerkleinerte Material wird bis zur Destraktion in einem Eisfach bei -20 °C gelagert.

Es werden zwei Destraktionen, Destraktion 1 und Destraktion 2, an einer Hochdruckextraktionsanlage durchgeführt. Die Hochdruckextraktionsanlage umfasst einen Extraktor, in welchem das Kohlenstoffdioxid in überkritischem Zustand vorliegt und das zu extrahierende Material extrahiert. Die Hochdruckextraktionsanlage umfasst zwei in Serie geschaltete Abscheider, Abscheider 1 und Abscheider 2.

In den Abscheidern herrscht eine niedrigere Temperatur und ein geringerer Druck als in dem Extraktor, wodurch sich der Extrakt abscheiden kann. Die Destraktion erfolgt in einem kontinuierlichen Modus, in welchem dem Extraktor kontinuierlich reines Kohlenstoffdioxid zugeführt wird und kontinuierlich mit Extrakt beladenes Kohlenstoffdioxid aus dem Extraktor in die Abscheider geführt wird. Bei der Überführung des mit Extrakt beladenen Kohlenstoffdioxides aus dem Extraktor in Richtung Abscheider wird das Gas expandiert und gekühlt. In dem Abscheider herrscht eine niedrigere Temperatur und ein geringerer Druck als in dem Extraktor, wodurch sich aus dem Kohlenstoffdioxid, welches aus dem Extraktor in den Abscheider überführt wird, ein Extrakt abscheidet. Nach Ablauf der Extraktionsdauer wird restliches Kohlenstoffdioxid über die Abscheider abgelassen. In Abscheider 1 hat sich als Extrakt eine gelbliche, nach Guave duftende Flüssigkeit abgeschieden (39 g bei Destraktion 1 bzw. 55 g bei Destraktion 2). Um die Trockenmasse zu bestimmen, wird der Extrakt im Trockenschrank bei 60 °C für 24 Stunden getrocknet. Die Trockenmasse der Extrakte beträgt 0,31 Gew.-% (Destraktion 1) bzw. 0,46 Gew.-% (Destraktion 2) und beschreibt den nichtflüchtigen Anteil. Die zur Destraktion verwendeten Parameter sind in Tabelle 1 zusammengefasst.

Tabelle 1

| Parameter | Destraktion | |
|---|---|---|
| | 1 | 2 |
| Extraktionsdauer [min] | 180 | 180 |
| Druck in dem Extraktor [bar] | 300 | 300 |
| Druck in dem Abscheider 1 [bar] | 45 | 45 |
| Druck in dem Abscheider 2 [bar] | 45 | 45 |
| Temperatur in dem Extraktor [°C] | 43 | 43 |
| Temperatur in dem Abscheider 1 [°C] | 25 | 25 |
| Temperatur in dem Abscheider 2 [°C] | 25 | 25 |
| Fluss an Kohlenstoffdioxid $CO_2$ [kg/h] | 20 | 20 |
| Gesamtverbrauch $CO_2$ [kg] | 61 | 60 |
| **Material** | | |
| Zu extrahierendes Material [g] | 2014 | 1860 |
| Cosolvens Wasser [g] | - | 186 |
| Rückstand im Extraktor [g] | 1981 | 1993 |
| Extrakt in Abscheider 1 [g] | 39 | 55 |
| Extrakt in Abscheider 2 [g] | - | - |
| Gesamtausbeute an Extrakt [Gew.-%] | 1,9 | 2,7 |
| Trockenmasse des Extraktes [Gew.-%] | 0,31 | 0,46 |

Vergleichsbeispiel, betreffend ein Verfahren zur Herstellung eines Extraktes aus Früchten der Echten Guave mittels ethanolischer Extraktion

Als zu extrahierendes Material, hergestellt aus Früchten der Echten Guave, wird "Single Strength White Guava Puree" von Tradework BV in Rotterdam in den Niederlanden bezogen. Das Mus weist einen Grad von mindestens 8,5 Brix auf, stammt aus Indien und wird, in einer Spezialfolie eingeschweißt, in einem gekühlten Aseptikfass geliefert. Das Mus weist keine Samen, Kerne oder Blätter der Echten Guave oder Teile davon auf. Das Mus wird auf Silikonmatten aufgebracht und im Trockenschrank bei 60 °C und unter regelmäßigem Wenden für 72 Stunden getrocknet. Durch Trocknung des Muses aus Früchten der Echten Guave wird ein Feststoff mit einem Wassergehalt von etwa 23 Gew.-% erhalten. Das zu extrahierende Material wird in einem Eisfach auf -20 °C gekühlt und dann mit einem JTC Blender zerkleinert, wobei die meisten Teilchen einen Durchmesser zwischen 0,1 mm und 10 mm aufweisen. Das zerkleinerte Material wird bis zur ethanolischen Extraktion in einem Eisfach bei -20 °C gelagert. 0,5 g des pulverförmigen Materiales werden in 25 mL Ethanol (80 Vol.-%) bei 60 °C 10 Minuten lang gerührt. Die Mischung wird filtriert und das Verfahren mit dem Rückstand wiederholt. Die Filtrate werden vereinigt, im Vakuum eingeengt und in 3 mL entionisiertem Wasser aufgenommen.

<u>Chemische Analyse der Extrakte</u>

Folgende Extrakte wurden per HPLC auf ihre Inhaltsstoffe untersucht:

- Die erfindungsgemäßen Extrakte aus Destraktion 1 und Destraktion 2,
- der ethanolische Vergleichsextrakt (nicht erfindungsgemäß),
- ein gereinigter und konzentrierter Extrakt aus Blättern der Echten Guave der Belan GmbH aus Wels in Österreich (nicht erfindungsgemäß),
- ein weiterer Extrakt aus Blättern der Echten Guave von Pfannenschmidt in Hamburg in Deutschland (nicht erfindungsgemäß) und
- ein Apfelextrakt von Pfannenschmidt in Hamburg in Deutschland (nicht erfindungsgemäß).

Zur Identifizierung von Polyphenolen wird bei den nicht erfindungsgemäßen Extrakten ein Agilent 1260 Infinity LC System mit Vakuumentgaser, quaternärer Pumpe, Autosampler und Photodiodenzeilendetektor verwendet. Hochauflösende Massenspektren werden mit einer Thermo Fisher Scientific LTQ Orbitrap XL mit einer Ion Max API Electrospray Source in negativem Ionisationsmodus erhalten. Folgende Parameter werden während der Analyse verwendet: Kapillartemperatur 350 °C, Hülsen-Gasfluss 45 U, Aux-Gasfluss 15 U, Quellenspannung 3,5 kV, Kapillarspannung -25 V, Tubus -90 V. Die Trennungen werden mit einer Hypersil ODS C18 Säule (250 mm x 4,6 mm Innendurchmesser, 5 $\mu$m Partikelgröße von Thermo Fisher Scientific, Wien in Österreich) durchgeführt. Analyten werden durch Gradientenelution mit 0,1 % Ameisensäure in Wasser (A) und Acetonitril, enthaltend 0,1 % Ameisensäure, (B) getrennt. Das Injektionsvolumen ist für alle Proben 5 $\mu$L und die Flussrate liegt bei 0,67 mL/min mit einem Gradienten für eine konstante Elution.

Zur Identifizierung von Polyphenolen wird bei den erfindungsgemäßen Extrakten aus Destraktion 1 und Destraktion 2 eine Thermo Scientific Dionex Ultimate 3000 mit LPG-3400SD Pumpe mit eingebautem Entgaser, gekühltem Autosampler WPS-3000 U(T)SL, temperaturkontrolliertem Säulenkompartiment und FLD-34000RS Photodiodenzeilendetektor mit Chromeleon-Software verwendet. Die Analyse erfolgt mit einer Accucore C18 Säule (150 mm x 3,0 mm Innendurchmesser, 2,6 $\mu$m Partikelgröße; Thermo Scientific). Die Säulentemperatur wird auf 40 °C eingestellt und das Injektionsvolumen beträgt 1 $\mu$L. Die Detektion erfolgt bei Wellenlängen von 260 nm und 360 nm. Die Analyten werden durch Gradientenelution bei einer Flußrate von 0,5 mL/min aufgetrennt, wobei die mobile Phase A 0.1 % Ameisensäure (FA) in Wasser enthält und die mobile Phase B 0.1 % FA in Acetonitril enthält. Zu Beginn werden 95 % von mobiler Phase A und 5 % von mobiler Phase B eingesetzt. Bei Minute 8 wird der Anteil von B auf 20 %, bei Minute 12 min auf 40 %, bei Minute 15 auf 60 % und bei Minute 17 auf 80 % erhöht. Bei Minute 20 wird der Anteil von B für weitere 5 Minuten auf 5 % gesenkt.

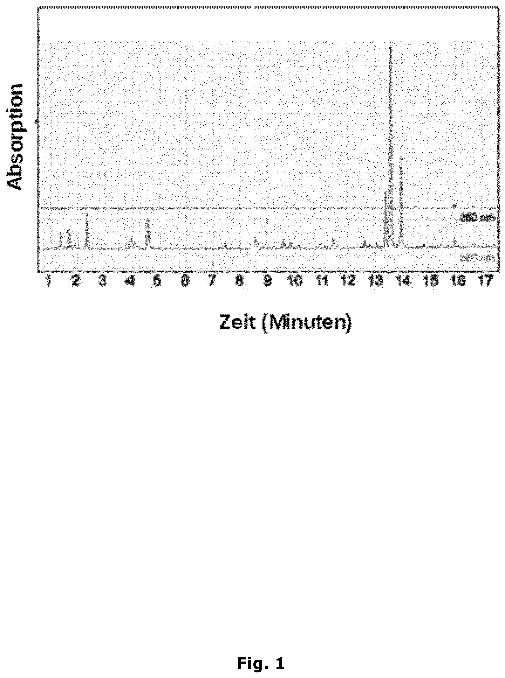

In den verschiedenen nicht erfindungsgemäßen Extrakten aus der Echten Guave können bis zu 16 verschiedene polyphenolische Verbindungen identifiziert werden: Phloridzin, Phloretin, Quercetin, Quercitrin, Isoquercitrin, Hyperosid, Avicularin, Guaijaverin, Procyanidin B1 und B2, (+)-Catechin, (-)-Catechin, (-)-Epicatechin, Gallocatechin, Epicatechingallat, Gallussäure und Ellagsäure. Allerdings wird keine dieser Verbindungen in den erfindungsgemäßen Extrakten aus Destraktion 1 und Destraktion 2 identifiziert. Ein HPLC-Chromatogramm eines erfindungsgemäßen Extraktes ist in Fig. 1 abgebildet.

Mit einer weiteren HPLC-Methode wurden die identifizierten Inhaltsstoffe der nicht erfindungsgemäßen Extrakte quantifiziert.

<u>Untersuchung der Hemmung des Glucosetransportes an Caco-2 Zellen</u>

Die Hemmung wurde an folgenden Extrakten untersucht:

- Erfindungsgemäßem Extrakt,
- ethanolischem Vergleichsextrakt (nicht erfindungsgemäß),

- gereinigtem und konzentriertem Extrakt aus Blättern der Echten Guave der Belan GmbH aus Wels in Österreich (nicht erfindungsgemäß),
- einem weiteren Extrakt aus Blättern der Echten Guave von Pfannenschmidt in Hamburg in Deutschland (nicht erfindungsgemäß).

Versuchsdurchführung:

Das Medium differenzierter Caco-2 Zellen wurde entfernt und die Zellen zweimal mit HEPES Puffer (20 mM HEPES, 137 mM NaCl, 4,7 mM KCl, 1,2 mM $MgSO_4$, 1,8 mM $CaCl_2$) gewaschen und in einer neuen Platte mit 12 Wells, enthaltend in dem basolateralen Kompartiment 800 µL HEPES-Puffer, platziert. Das apikale Kompartiment wurde dann mit 500 µL Donorlösung, enthaltend Zellkulturmedium mit 2,1 g/L Glucose, 1,0 g/L Xylitol und einen Extrakt, aufgefüllt. In Kontrollversuchen wurde 500 µL Donorlösung, enthaltend Zellkulturmedium mit 2,1 g/L Glucose und 1,0 g/L Xylitol verwendet. Proben von 100 µL wurden dem basolateralen Kompartiment zu bestimmten Zeitpunkten entnommen und mittels HPLC auf ihren Glucosegehalt untersucht. TEER Werte wurden zu jedem Zeitpunkt untersucht, um die Integrität der Schicht aus einzelnen Zellen sicherzustellen. Schließlich wurden 100 µL der Donorlösung aus dem apikalen Kompartiment verwendet, um die übrige Glucosekonzentration zu quantifizieren. Zur Behandlung der Extrakte mit Verdauungssäften wurden 100 mg jedes Extraktes 2 Stunden bei 37 °C mit 1 mL Darmsaft (KCl 0,3 g/L, $CaCl_2$ 0,5 g/L, $MgCl$ 0,2 g/L, $NaHCO_3$ 1 g/L, Trypsin 0,3 g/L, Pankreatin 9 g/L, Harnstoff 0,3 g/L, pH 7,5) behandelt. Dann wurden die Proben mit einem Kulturmedium mit 2,1 g/L Glucose und 1,0 g/L Xylitol auf eine finale Konzentration von 100 mg/L bzw. mL/L verdünnt. Die Zuckeranalyse erfolgte mit einem Jasco LC-2000 Plus Series System, umfassend eine analytische Pumpe mit externem Entgaser, Autosampler, temperaturkontrollierbarem Säulenkompartiment, einem Jasco RI-2031 Plus Detektor und einem UV-Vis Detektor, ausgerüstet mit Chrompass Software (alles von der Jasco Corpoation, Tokyo, Japan). Die Trennung erfolgte auf folgender Säule: Varian, Meta Carb 87H (PN A5210, SN 12509907). Die Säulentemperatur wurde auf 56 °C eingestellt und eine isokratische Elution wurde bei 0,8 mL/min durchgeführt. Als mobile Phase wurde 6 mM Schwefelsäure in doppelt entionisiertem Wasser verwendet. Daten wurden mit Jasco Chrompass Chromatography System Software verarbeitet.

Ergebnisse:

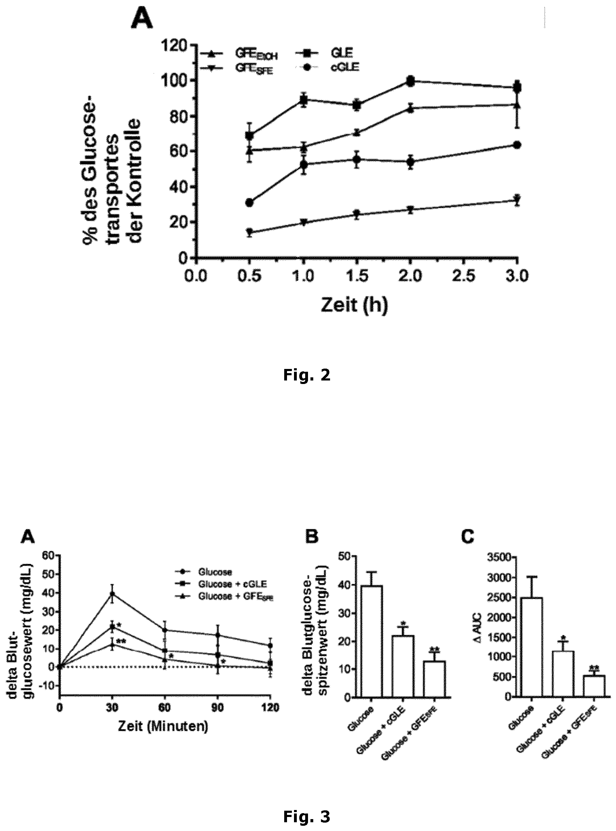

Die Ergebnisse zur Hemmung des Glucosetransportes von Caco-2 Zellen durch die Extrakte sind in Fig. 2 dargestellt. Abgebildet ist der in Gegenwart der Extrakte stattfindende Glucosetransport durch die Caco-2 Zellen hindurch. 100 % Glucosetransport entspricht dem, in den Kontrollversuchen ohne Extrakte stattfindendem, ungehemmten Glucosetransport. Die Hemmung des Glucosetransportes ist durch alle Extrakte bei 30 Minuten am ausgeprägtesten und nimmt über die Stunden ab. Die stärkste Hemmung des Glucosetransportes erfolgt durch den erfindungsgemäßen Extrakt ($GFE_{SFE}$, bei 30 Minuten nur ca. 13 % des Glucosetransportes der Kontrolle). Eine starke Hemmung erfolgt auch durch den gereinigten und konzentrierten Extrakt aus Blättern der Echten Guave (cGLE, bei 30 Minuten nur ca. 30 % des Glucosetransportes der Kontrolle). Der ethanolische Vergleichsextrakt ($GFE_{EtOH}$) und der weitere Extrakt aus Blättern der Echten Guave (GLE) zeigen bei 30 Minuten ca. 60 % bis 70 % des Glucosetransportes der Kontrolle.

Untersuchung der Hemmung intestinaler Glucose-Resorption und der Reduzierung von Blutzuckerspitzen im Tierversuch

Die Wirkung folgender Extrakte wurde untersucht:

- Erfindungsgemäßer Extrakt,
- gereinigter und konzentrierter Extrakt aus Blättern der Echten Guave der Belan GmbH aus Wels in Österreich (nicht erfindungsgemäß).

Versuchsdurchführung:

Weibliche C57BL/6N Mäuse wurden im Alter von acht Wochen von den Janvier Labs erhalten und konventionell bei 22 °C, einem Zyklus mit 12 Stunden Licht und 12 Stunden Dunkelheit und freiem Zugang zu Wasser und Nahrung (Ratten/Mäuse Erhaltungsfutter, 10 mm, V1534-000, Ssniff GmbH, Deutschland) gehalten. Die Mäuse wurden zu Beginn in zwei Gruppen, bestehend aus jeweils sechs Mäusen, aufgeteilt. Damit die Tiere sich an die lokale Umgebung gewöhnen, wurden die Mäuse im Tierraum mindestens 24 Tage ohne Behandlung gehalten. Am Tag bevor die oralen Glucosetoleranztests (OGTTs) durchgeführt wurden, wurden die Mäuse in den Versuchsraum gebracht und 12 Stunden lang, abgesehen von Wasser, von Nahrung ferngehalten. Testlösungen wurden an jedem Tag frisch hergestellt. Die oralen Glucosetoleranztests wurden an zwei Tagen durchgeführt, mit sechs Mäusen pro Testtag in den folgenden Gruppen:

Kontrollgruppe (1,5 g Glucose/kg Körpergewicht), Blätterextraktgruppe (1,5 g Glucose + 400 mg des gereinigten und konzentrierten Extraktes aus Blättern der Echten Guave /kg Körpergewicht) und Gruppe für erfindungsgemäßen Extrakt (1,5 g Glucose + 400 mg erfindungsgemäßer Extrakt /kg Körpergewicht). Die Testlösungen wurden oral per Sondenfütterung verabreicht und Blutproben wurden aus der Schwanzspitze vor und 30, 60, 90 und 120 Minuten nach Glucoseverabreichung genommen. Die Blutproben wurden mit einem Glucometer Contour XT Instument von Bayer, Deutschland, auf den Glucosegehalt untersucht. Die Experimente wurden vom österreichischen Tierethikkommitee genehmigt.

Ergebnisse:

Die Ergebnisse sind in Fig. 3A, 3B und 3C dargestellt. Fig. 3A zeigt den relativen Anstieg (delta) der Blutglucosewerte über die Dauer von 120 Minuten. Als Referenzwert für den relativen Anstieg dienen die Blutglucosewerte vor der oralen Glucosegabe. Bei der Gabe reiner Glucose kommt es bei Minute 30 zu einem starken Anstieg des Blutglucosewertes (Fig. 3A und 3B). Bei Gabe von Glucose und erfindungsgemäßem Extrakt ($GFE_{SFE}$) ist der Anstieg bei Minute 30 deutlich geringer. Eine etwas weniger starke Wirkung entfaltet der gereinigte und konzentrierte Extrakt aus Blättern der Echten Guave (cGLE). In allen Gruppen tritt also 30 Minuten nach oraler Glucosegabe eine Blutzuckerspitze auf, wobei der Blutglucoseanstieg in der Gruppe mit beigemischtem, erfindungsgemäßem Extrakt am geringsten ist (delta 12,6 ± 3,3 mg/dL), gefolgt von der Gruppe mit dem Vergleichsextrakt (delta 21,8 ± 3,2 mg/dL). In der Kontrollgruppe wird der höchste Blutglucosewert (delta 39,5 ± 4,9 mg/dL) erreicht. Der erfindungsgemäße Extrakt vermeidet also Blutzuckerspitzen. Fig. 3C bildet die Fläche unter den jeweiligen Kurven der Fig. 3A ab. Diese Flächen bilden genähert die innerhalb der zwei Stunden insgesamt intestinal resorbierte Glucose ab (delta AUC). Der erfindungsgemäße Extrakt führt im Vergleich zur reinen Glucoselösung und zur Glucoselösung mit dem anderen Extrakt zu einer deutlich reduzierten Menge intestinal resorbierter Glucose.

Verwendung des Extraktes bzw. einer Zusammensetzung, enthalten den Extrakt

Es wurde gezeigt, dass der erfindungsgemäße Extrakt bzw. eine Zusammensetzung, enthaltend den Extrakt, die Reduzierung von Blutzuckerspitzen und die Hemmung intestinaler Glucose-Resorption ermöglicht. Durch die Hemmung der intestinalen Glucose-Resorption eignet sich der Extrakt bzw. eine Zusammensetzung, enthalten den Extrakt, auch zur Gewichtsreduktion. Somit empfiehlt sich der erfindungsgemäße Extrakt bzw. eine Zusammensetzung, enthaltend den Extrakt, sowohl zur Verwendung als Nahrungsergänzungsmittel als auch als Arzneimittel.

Nahrungsergänzungsmittel

Für die Verwendung als Nahrungsergänzungsmittel bietet es sich an, den Extrakt als Lösung des Extraktes in wässrigem Ethanol oder Propanol, auf eine Trägersubstanz, z.B. Lactose, Maltodextrin, Carbonat, Algenpulver und/oder Guarkernmehl, die währenddessen durchmischt wird, aufzutragen, insbesondere aufzusprühen. Das erhaltene Material wird homogenisiert, im Trockenschrank getrocknet, mit weiteren Substanzen zur Bildung eines Nahrungsergänzungsmittels vermischt und beispielsweise in Kapseln abgefüllt. Nachfolgend sind die weiteren Zutaten eines Ausführungsbeispieles aufgeführt, bei dem es sich um ein Nahrungsergänzungsmittel in Pulverform bzw. Kapselform handelt. Das Nahrungsergänzungsmittel wird mit einer Mahlzeit eingenommen. Die Zutaten sind insbesondere Fruktose, Gummi Arabicum, Haferkleie, Erbsenfasern, Verdickungsmittel (Guarkernmehl, Pektin), Säuerungsmittel (Zitronensäure), Guaranaextraktpulver (Maltodextrin, Guaranaextrakt, Koffein), Aroma, Acerolaextraktpulver (Maltodextrin, Vitamin C, Acerolaextrakt), Vitamin C, Rote Bete Pulver (Rote-Bete Saftkonzentrat, Maltodextrin, Säureregulator (Citronensäure)), Apfelfaser, Gemüsepulver (Brokkoli, Weißkohl, Karotten (Maltodextrin, Karotten), Paprika, Spinat, Tomaten), Multienzymkomplex (Amylase, Lactase, Protease, Cellulase, Lipase), Inulin, Reiskleie, Milchsäurekulturen (Lactobacillus acidophilus, Laciobacillus reuteri), Kurkuma-Extrakt mit gamma-Cyclodextrin, Süßungsmittel (Steviolglycoside (Steviolglycoside, Aroma)), Selenhefe, Niacin, Grünteeextrakt, Algenpulver, Vitamin E, Beta-Carotin, Pantothensäure, Traubenkernextrakt, Vitamin B6, Vitamin B2, Vitamin B1, Folsäure, Biotin und Vitamin B12. Weitere Nährwertangaben des Nahrungsergänzungsmittels sind in nachfolgender Tabelle 2 wiedergegeben, wobei sich die jeweiligen Angaben auf 100 g des Nahrungsergänzungsmittels beziehen:

Tabelle 2

| | |
|---|---|
| Fett | < 0,4 g |
| Kohlenhydrate | 53 g |
| Ballaststoffe | 33 g |

(fortgesetzt)

| | |
|---|---|
| Eiweiß | 1,0 g |
| Salz (Natriumchlorid) | < 0,3 g |
| Selen | 200 µg |
| Vitamin C | 1000 mg |
| Niacin | 340 mg NE |
| Vitamin E | 67 mg alpha-TE |
| Pantothensäure | 60 mg |
| Vitamin B1 | 14 mg |
| Vitamin B2 | 16 mg |
| Vitamin B6 | 20 mg |
| Vitamin B12 | 10 µg |
| Vitamin A (aus Provitamin A) | 2220 µg RE |
| Folsäure | 2000 µg |
| Biotin | 1500 µg |
| Koffein, insbesondere aus Guaranaextraktpulver | 200 mg |

Arzneimittel

Die Reduzierung von Blutzuckerspitzen, die Hemmung intestinaler Glucose-Resorption und Gewichtsreduktion sind wichtige Ziele bei der Behandlung und Prävention von Diabetes mellitus, Hyperglykämie und Adipositas. Daher empfiehlt sich der erfindungsgemäße Extrakt bzw. eine Zusammensetzung, enthalten den Extrakt, zur Verwendung bei der Behandlung und Prävention von Diabetes mellitus und/oder Hyperglykämie und/oder Adipositas. Das Arzneimittel wird oral mit den Mahlzeiten eingenommen, um die Auswirkungen in der Nahrung vorliegender Zucker zu mildern und unterstützt eine Therapie mit Insulin, Acarbose, Biguaniden, Gliniden, DPP-IV-Inhibitoren, Glitazonen und/oder Sulfonylharnstoffen. Für die Verwendung als Arzneimittel bietet es sich an, den Extrakt als Lösung des Extraktes in wässrigem Ethanol oder Propanol, auf eine Trägersubstanz, z.B. Lactose, Maltodextrin, Carbonat, Algenpulver und/oder Guarkernmehl, die währenddessen durchmischt wird, aufzutragen, insbesondere aufzusprühen. Das Material wird homogenisiert, im Trockenschrank getrocknet, optional mit weiteren Substanzen gemischt und beispielsweise in Kapseln abgefüllt. Auch andere Darreichungsformen wie Tabletten, Lösungen, Emulsionen, orale Filme sind möglich.

**Patentansprüche**

1. Verfahren zur Herstellung eines Extraktes aus Früchten der Echten Guave (Psidium Guajava), umfassend:

   a) Bereitstellen eines zu extrahierenden Materiales, hergestellt aus Früchten der Echten Guave;
   b) Destraktion des zu extrahierenden Materiales mit Kohlenstoffdioxid als Destraktionsmittel in einer Hochdruckextraktionsanlage, um einen Extrakt zu erhalten, wobei dem zu extrahierenden Material für die Destraktion Wasser als Cosolvens zugesetzt wird.

2. Verfahren nach Anspruch 1, wobei das zu extrahierende Material ein Mus aus Früchten der Echten Guave oder einen Feststoff, erhalten durch Trocknung eines Muses aus Früchten der Echten Guave, umfasst.

3. Verfahren nach Anspruch 1 oder 2, wobei das zu extrahierende Material vor der Destraktion auf einen Wassergehalt von 3 Gew.-% bis 50 Gew.-%, bevorzugt von 5 Gew.-% bis 35 Gew.-%, besonders bevorzugt von 7 Gew.-% bis 30 Gew.-%, getrocknet wird.

4. Verfahren nach einem der vorherigen Ansprüche, wobei das zu extrahierende Material vor der Destraktion zu einem Feststoff mit einem Wassergehalt von 7 Gew.-% bis 30 Gew.-% getrocknet wird.

**5.** Verfahren nach einem der vorherigen Ansprüche, wobei dem zu extrahierenden Material für die Destraktion als Cosolvens 1 Gew.-% bis 30 Gew.-% Wasser, besonders bevorzugt 8 Gew.-% bis 20 Gew.-% Wasser, zugesetzt wird, bezogen auf die Trockenmasse des zu extrahierenden Materiales.

**6.** Verfahren nach einem der vorherigen Ansprüche, wobei der Druck in einem Extraktor der Hochdruckextraktionsanlage während der Destraktion 100 bar bis 500 bar, bevorzugt 200 bar bis 400 bar, ganz besonders bevorzugt 250 bar bis 350 bar, beträgt und/oder die Temperatur in einem Extraktor der Hochdruckextraktionsanlage während der Destraktion 31 °C bis 80 °C, bevorzugt 31 °C bis 50 °C, beträgt.

**7.** Verfahren nach einem der vorherigen Ansprüche, wobei der erhaltene Extrakt von den Verbindungen Phloridzin, Phloretin, Quercetin, Quercitrin, Isoquercitrin, Hyperosid, Avicularin, Guaijaverin, Procyanidin B1 und B2, (+)-Catechin, (-)-Catechin, (-)-Epicatechin, Gallocatechin, Epicatechingallat, Gallussäure und Ellagsäure jeweils weniger als 0,8 Gew.-%, bevorzugt jeweils weniger als 0,3 Gew.- %, bezogen auf die Trockenmasse des Extraktes, enthält.

**8.** Verfahren nach einem der vorherigen Ansprüche, wobei die Destraktion über 1 h bis 8 h, bevorzugt 2 h bis 4 h, erfolgt, und/oder die Hochdruckextraktionsanlage einen Extraktor und einen Abscheider umfasst und der Extraktor während der Destraktion durch einen kontinuierlichen Fluss an Kohlenstoffdioxid von 2 bis 20, bevorzugt von 5 bis 12, Kilogramm Kohlenstoffdioxid pro Stunde pro Kilogramm zu extrahierenden Materiales, durchströmt wird, wobei der kontinuierliche Fluss an Kohlenstoffdioxid dem Abscheider zugeführt wird.

**9.** Verfahren nach einem der vorherigen Ansprüche, wobei in einem Abscheider der Hochdruckextraktionsanlage während der Destraktion eine Temperatur von 10 °C bis 40 °C, bevorzugt von 15 °C bis 35 °C, vorliegt und/oder ein Druck von 15 bar bis 55 bar, bevorzugt von 30 bar bis 50 bar, vorliegt.

**10.** Extrakt, erhältlich durch das Verfahren nach einem der Ansprüche 1 bis 9.

**11.** Zusammensetzung, enthaltend den Extrakt nach Anspruch 10.

**12.** Zusammensetzung nach einem der Ansprüche 10 oder 11, insbesondere 11, zur Verwendung als Nahrungsergänzungsmittel und/oder Arzneimittel.

**13.** Zusammensetzung nach Anspruch 12 zur Verwendung zur Reduzierung von Blutzuckerspitzen und/oder zur Hemmung intestinaler Glucose-Resorption und/oder zur Gewichtsreduktion.

**14.** Zusammensetzung nach Anspruch 12 oder 13 zur Verwendung bei der Behandlung oder Prävention von Diabetes mellitus und/oder Hyperglykämie und/oder Adipositas.

**15.** Verfahren zur Herstellung einer Zusammensetzung nach Anspruch 11, insbesondere eines Nahrungsergänzungsmittels, umfassend:

a) Bereitstellen eines Extraktes, erhältlich durch das Verfahren nach einem der Ansprüche 1 bis 10;
b) Auftragen des Extraktes auf eine Trägersubstanz, insbesondere durch Aufsprühen einer Lösung des Extraktes auf eine Trägersubstanz, um eine extrakthaltige Trägersubstanz zu erhalten;
c) Mischen der extrakthaltigen Trägersubstanz mit weiteren Bestandteilen der Zusammensetzung, insbesondere des Nahrungsergänzungsmittels.

**Claims**

**1.** Method for producing extracts from fruits of the true guava (Psidium guajava), comprising:

a) providing a material to be extracted, produced from fruits of the true guava;
b) extracting by supercritical fluid the material to be extracted using carbon dioxide as a supercritical fluid extraction agent and a high-pressure extraction system to obtain an extract, wherein water is added as a co-solvent to the material to be extracted by supercritical fluid.

**2.** Method as claimed in claim 1, wherein the material to be extracted comprises a pulp made of fruits of the true guava or a solid obtained by drying a pulp made of fruits of the true guava.

3. Method as claimed in claim 1 or 2, wherein the material to be extracted is dried before the supercritical fluid extraction to a water content of 3 wt.% to 50 wt.%, preferably of 5 wt.% to 35 wt.%, particularly preferably of 7 wt.% to 30 wt.%.

4. Method as claimed in any one of the preceding claims, wherein the material to be extracted is dried before the supercritical fluid extraction to form a solid having a water content of 7 wt.% to 30 wt.%.

5. Method as claimed in any one of the preceding claims, wherein 1 wt.% to 30 wt.% water, particularly preferably 8 wt.% to 20 wt.% water, in relation to the dry mass of the material to be extracted, is added as a co-solvent to the material to be extracted for the supercritical fluid extraction.

6. Method as claimed in any one of the preceding claims, wherein the pressure in an extractor of the high-pressure extraction system during the supercritical fluid extraction is 100 bar to 500 bar, preferably 200 bar to 400 bar, very particularly preferably 250 bar to 350 bar, and/or the temperature in an extractor of the high-pressure extraction system during the supercritical fluid extraction is 31°C to 80°C, preferably 31°C to 50°C.

7. Method as claimed in any one of the preceding claims, wherein the extract obtained contains less than 0.8 wt.%, preferably less than 0.3 wt.%, in relation to the dry mass of the extract, of each of the compounds phloridzin, phloretin, quercetin, quercitrin, isoquercitrin, hyperosid, avicularin, guaijaverin, procyanidin B1 and B2, (+)-catechin, (-)-catechin, (-)-epicatechin, gallocatechin, epicatechin gallate, gallic acid, and ellagic acid.

8. Method as claimed in any one of the preceding claims, wherein the supercritical fluid extraction takes place over 1 hour to 8 hours, preferably 2 hours to 4 hours, and/or the high-pressure extraction system comprises an extractor and a separator and a continuous flow of carbon dioxide of 2 to 20, preferably of 5 to 12, kilograms of carbon dioxide per hour per kilogram of material to be extracted flows through the extractor during the supercritical fluid extraction, wherein the continuous flow of carbon dioxide is supplied to the separator.

9. Method as claimed in any one of the preceding claims, wherein a temperature of 10°C to 40°C, preferably of 15°C to 35°C, and/or a pressure of 15 bar to 55 bar, preferably of 30 bar to 50 bar is present in a separator of the high-pressure extraction system during the supercritical fluid extraction.

10. Extract obtainable by the method as claimed in any one of claims 1 to 9.

11. Composition containing the extract as claimed in claim 10.

12. Composition as claimed in either one of claims 10 or 11, in particular 11, for use as a nutritional supplement and/or medication.

13. Composition as claimed in claim 12 for use for reducing blood sugar spikes and/or for inhibiting intestinal glucose resorption and/or for reducing weight.

14. Composition as claimed in claim 12 or 13 for use in the treatment or prevention of diabetes mellitus and/or hyperglycemia and/or obesity.

15. Method for producing a composition as claimed in claim 11, in particular a nutritional supplement, comprising:

a) providing an extract obtainable by the method as claimed in any one of claims 1 to 10;
b) applying the extract to a carrier substance, in particular by spraying a solution of the extract onto a carrier substance, to obtain an extract-containing carrier substance;
c) mixing the extract-containing carrier substance with further components of the composition, in particular of the nutritional supplement.

**Revendications**

1. Procédé pour la fabrication d'un extrait de fruits de goyavier vrai (*Psidium guajava*), comprenant :

a) la préparation d'une matière qui doit être soumise à l'extraction, fabriquée à partir de fruits du goyavier vrai ;
b) extraction par gaz liquéfié de la matière qui doit être soumise à l'extraction avec du dioxyde de carbone servant d'agent d'extraction dans une installation d'extraction sous haute pression afin d'obtenir un extrait, la matière qui doit être soumise à l'extraction étant additionnée d'eau qui sert de co-solvant pour l'extraction par gaz liquéfié.

2. Procédé selon la revendication 1, dans lequel la matière qui doit être soumise à l'extraction est une purée de fruits du goyavier vrai ou un solide obtenu par séchage d'une purée de fruits du goyavier vrai.

3. Procédé selon la revendication 1 ou 2, dans lequel la matière qui doit être soumise à l'extraction est séchée avant l'extraction par gaz liquéfié jusqu'à une teneur en eau de 3 % en poids à 50 % en poids, de préférence de 5 % en poids à 35 % en poids, en particulier de 7 % en poids à 30 % en poids.

4. Procédé selon l'une des revendications précédentes, dans lequel la matière qui doit être soumise à l'extraction est séchée avant l'extraction par gaz liquéfié afin d'obtenir un solide ayant une teneur en eau de 7 % en poids à 30 % en poids.

5. Procédé selon l'une des revendications précédentes, dans lequel la matière qui doit être soumise à l'extraction est additionnée, en vue de l'extraction par gaz liquéfié, de 1 % en poids à 30 % en poids d'eau servant de co-solvant, en particulier de 8 % en poids à 20 % en poids d'eau, par rapport à la masse sèche de la matière qui doit être soumise à l'extraction.

6. Procédé selon l'une des revendications précédentes, dans lequel la pression dans un extracteur de l'installation d'extraction sous haute pression pendant l'extraction par gaz liquéfié est de 100 bars à 500 bars, de préférence de 200 bars à 400 bars, tout particulièrement de 250 bars à 350 bars, et/ou la température dans un extracteur de l'installation d'extraction sous haute pression pendant l'extraction par gaz liquéfié est de 31 °C à 80 °C, de préférence de 31 °C à 50 °C.

7. Procédé selon l'une des revendications précédentes, dans lequel l'extrait obtenu contient respectivement moins de 0,8 % en poids, de préférence respectivement moins de 0,3 % en poids, par rapport à la masse sèche de l'extrait, des composés suivants : phloridzine, phlorétine, quercétine, quercitrine, isoquercitrine, hypéroside, avicularine, guaijavérine, procyanidine B1 et B2, (+)-catéchine, (-)-catéchine, (-)-épicatéchine, gallocatéchine, gallate d'épicatéchine, acide gallique et acide ellagique.

8. Procédé selon l'une des revendications précédentes, dans lequel l'extraction par gaz liquéfié se déroule sur 1 h à 8 h, de préférence 2 h à 4 h, et/ou l'installation d'extraction sous haute pression comprend un extracteur et un séparateur et l'extracteur est parcouru pendant l'extraction par gaz liquéfié par une circulation continue de dioxyde de carbone de 2 à 20, de préférence de 5 à 12 kilogrammes de dioxyde de carbone par heure par kilogramme de matière qui doit être soumise à l'extraction, la circulation continue de dioxyde de carbone étant amenée au séparateur.

9. Procédé selon l'une des revendications précédentes, dans lequel une température de 10 °C à 40 °C, de préférence de 15 °C à 35 °C, et/ou une pression de 15 bars à 55 bars, de préférence de 30 bars à 50 bars, règnent dans un séparateur de l'installation d'extraction sous haute pression pendant l'extraction par gaz liquéfié.

10. Extrait pouvant être obtenu par le procédé selon l'une des revendications 1 à 9.

11. Composition contenant l'extrait selon la revendication 10.

12. Composition selon l'une des revendications 10 ou 11, en particulier 11, destinée à être utilisée comme complément alimentaire et/ou comme médicament.

13. Composition selon la revendication 12, destinée à être utilisée pour réduire les pics de glycémie et/ou inhiber l'absorption intestinale du glucose et/ou en vue d'une réduction pondérale.

14. Composition selon la revendication 12 ou 13, destinée à être utilisée dans le traitement ou la prévention du diabète sucré et/ou de l'hyperglycémie et/ou de l'obésité.

15. Procédé pour la fabrication d'une composition selon la revendication 11, en particulier d'un complément alimentaire, comprenant :

a) la préparation d'un extrait pouvant être obtenu par le procédé selon l'une des revendications 1 à 10 ;

b) l'application de l'extrait sur une substance véhicule, en particulier par pulvérisation d'une solution de l'extrait sur une substance véhicule afin d'obtenir un véhicule contenant l'extrait ;

c) mélange du véhicule contenant l'extrait avec d'autres composants de la composition, en particulier du complément alimentaire.

Fig. 1

**Fig. 2**

**Fig. 3**

**IN DER BESCHREIBUNG AUFGEFÜHRTE DOKUMENTE**

**In der Beschreibung aufgeführte Patentdokumente**

- WO 2017137405 A
- US 2010249248 A1
- CN 104984186 A
- CN 106107993 A

**In der Beschreibung aufgeführte Nicht-Patentliteratur**

- **CASTRO-VARGAS H. I. et al.** *J. of Supercritical Fluids*, 2010, vol. 51, 319-324
- **MOURA P. M. et al.** *J. of Supercritical Fluids*, 2012, vol. 62, 116-122